# EUROPEAN PATENT APPLICATION

(11) **EP 3 165 604 A1**
(43) Date of publication of application: **10.05.2017**
(21) Application number: 15193102.9
(22) Date of filing: 05.11.2015
(51) Int. Cl.: C12N 7/02

(54) **METHOD FOR THE PURIFICATION OF VIRUS COMPOSITIONS AND VIRUS COMPOSITIONS OBTAINED**

(71) Applicant: Max-Planck-Gesellschaft zur Förderung der Wissenschaften e.V., 80539 München (DE)
(72) Inventor: WOLFF, Michael, 39175 Biederitz (DE); PIELER, Michael Martin, 39104 Magdeburg (DE); REICHL, Udo, 39104 Magdeburg (DE); MARICHAL-GALLARDO, Pavel, 39114 Magdeburg (DE)
(74) Representative: Kröncke, Rolf

(57) **Abstract**

In a first aspect, the present invention relates to a method for the purification of virus compositions as well as biological macromolecular compounds in a sample comprising mixing the sample with non-ionic organic polymers and contacting the mixed sample with a hydrophilic membrane, optionally washing the membrane, and eluting the virus preparations or biological macromolecular components from the membrane with an eluting solution containing reduced amounts or no non-ionic organic polymer. Moreover, virus compositions and biological macromolecular components obtainable with the method according to the present invention are provided as well as the use of the method according to the present invention for purification of virus compositions including whole virus particles and virus-like particles or biological macromolecular components.

## Description

In a first aspect, the present invention relates to a method for the purification of virus compositions as well as biological macromolecular components in a sample comprising mixing the sample with non-ionic organic polymers and contacting the mixed sample with a hydrophilic membrane, optionally washing the membrane, and eluting the virus preparations or biological macromolecular components from the membrane with an eluting solution containing reduced amounts or no non-ionic organic polymer. Moreover, virus compositions and biological macromolecular components obtainable with the method according to the present invention are provided as well as the use of the method according to the present invention for purification of virus compositions including whole virus particles and virus-like particles or biological macromolecular components.

### Background art

Isolation of whole virus particles, viral vectors, and virus-like particles (VLPs) as well as virus proteins, in the following referred to as virus, is of most importance for the production of vaccines useful in prophylactic or therapeutic vaccines against viral infections or for viral vectors used in gene therapy. The downstream processing of viruses obtained from natural resources, including egg- or cell culture-based production systems, is an elaborate process that requires careful design consideration. Virus-like particles resemble viruses, but are non-infectious because they do not contain any viral genetic material. The expression of viral structural proteins, such as envelope or capsule proteins, can result in the self-assembly of virus-like particles. Viruses as viral vectors are also in the focus of gene therapy.

The degree of complexity for downstream processing of viruses is related to the product source to be purified including the initial volume, product concentration, concentration and heterogeneity of impurities as well as the product itself including its physiochemical characteristics, stability, the final amount required, and the form to be provided.

Various methods have been described for enabling downstream processing for obtaining purified virus material. For example, EP 2 144 937 B1 describes a method for purification of virus and viral proteins based on sulphated cellulose membranes.

Size exclusion chromatography (SEC) requires column packing and represents a cost extensive, laborious method for purification of virus compositions. Other methods are described in the art, including ion-exchange chromatography based on the use of monoliths.

Furthermore, in WO 2012/169970 processes are described for the purification of biological products by constrained co-hydration chromatography (steric-exclusion chromatography). The method described therein generally refers to the purification of hydrated target species including purification of biological materials such as antibodies, viruses, cells and cellular organelles in connection with chromatography matrices.

Recently, Gagnon P. et al, Journal of Chromatography A 2014, 1324, 171-180 described a high productivity purification of immunoglobulin G monoclonal antibodies on starch coated magnetic nanoparticles by steric exclusion with polyethylene glycol as an alternative for packed chromatography columns.

Despite the advantages monoliths offer, the use of monoliths is expensive when compared to membrane absorbers, at least for vaccine manufacture. Thus, the costs of the monoliths challenge their single use application. Hence, they require cleaning validation and the risk of process inconsistency due to fouling.

In addition, complex means are required for effecting traditional size exclusion chromatography as well as constrained co-hydration chromatography via monoliths.

That is, major drawbacks of applying column chromatography include long process time with large recovery liquid volume as well as laborious and complex process steps.

Therefore, it is an object of the present invention to provide a method for purification of virus compositions as well as biological macromolecular components in a simple manner overcoming the drawbacks in the art.

It is another object of the present invention to provide virus compositions, in particular, virus particles and virus like particles with high purity as well as biological macromolecular components.

### Summary of the present invention

In a first aspect, the present invention relates to a method for the purification of virus compositions and/or biological macromolecular components in a sample comprising steps of:
- mixing the sample containing virus compositions and/or biological macromolecular components with a non-ionic organic polymer;
- contacting the mixture of the sample containing virus composition and/or biological macromolecular components and non-ionic organic polymer with a hydrophilic membrane;
- optionally washing the hydrophilic membrane with a washing solution containing a non-ionic organic polymer; and
- eluting the virus composition and/or biological macromolecular components from the membrane with an elution solution containing a reduced concentration compared to the concentration present in the sample or no non-ionic organic polymer for obtaining purified virus compositions and/or biological macromolecular components.

Particularly preferred, the hydrophilic membrane is a cellulose membrane, like regenerated cellulose or a reinforced cellulose membrane, while in another embodiment, the non-ionic organic polymer is an aliphatic polyether like polyalkylene glycol, in particular a polyethylene glycol, or polypropylene glycol, or a poloxamer.

In another aspect, the present invention relates to the virus compositions and/or biological macromolecular components obtainable with the method according to the present invention. In a preferred embodiment, the virus composition is a purified virus composition of either whole virus particles or virus like particles.

In another aspect, the present invention provides the use of the method according to the present invention for isolating virus compositions and/or biological macromolecular components. Finally, the present invention provides the use of a kit for purification of virus particles and/or virus like particles as well as biological macromolecular components including non-ionic organic polymers as defined herein as well as a hydrophilic membrane. The kit is preferably designed for single use.

### Brief description of the drawings

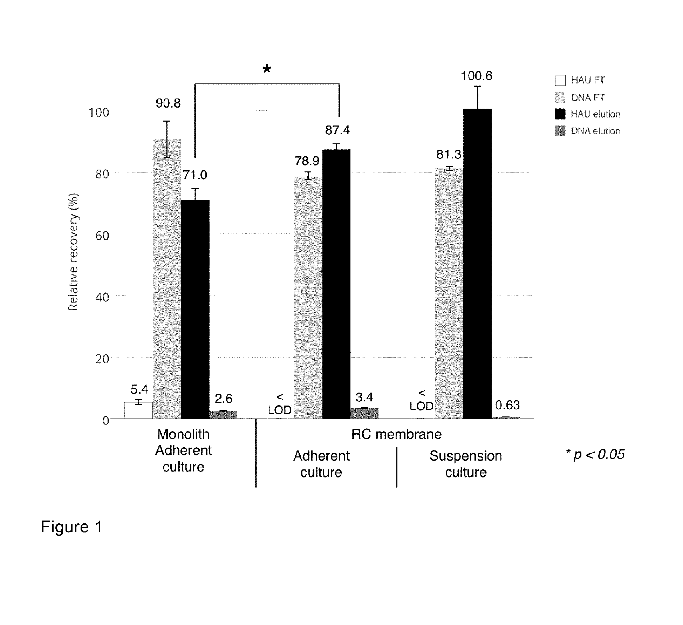

Figure 1 shows the results of the purification of MDCK cell culture-based influenza virus particles with hydroxyl (OH) monoliths and regenerated cellulose membranes. The different purifications were performed at the same experimental conditions, i.e. binding buffer and elution buffer. The best performance was achieved for MDCK suspension cell culture-derived influenza virus particles using regenerated cellulose membranes with no detectable hemagglutination activity (aHA) losses, expressed as hemagglutination units (HAU) in the collected product fraction with a DNA clearance higher than 99%. Relative recoveries and standard error of hemagglutination units (HAU) and DNA from the purification of adherent (MDCK_{adh}) and suspension (MDCKₛᵤₛ) cell culture-based influenza virus particles. FT= flowthrough, RC=regenerated cellulose.

### Detailed description of the present invention

In a first embodiment, the present invention relates to a method for the purification of virus compositions and/or biological macromolecular components from a sample comprising the steps of:
- mixing the sample containing virus compositions and/or biological macromolecular components with a non-ionic organic polymer;
- contacting the mixture of the sample containing virus composition and/or biological macromolecular components and non-ionic organic polymer with a hydrophilic membrane;
- optionaly washing the hydrophilic membrane with a washing solution containing a non-ionic organic polymer; and
- eluting the virus composition and/or biological macromolecular components from the membrane with an elution solution containing a reduced amount of the non-ionic organic polymer compared to the amount present in the sample or no a non-ionic organic polymer for obtaining purified virus compositions and/or biological macromolecular components.

That is, the present inventors recognized that by simply adding the non-ionic polymer to the sample containing virus compositions and/or biological macromolecular components and, after mixing the sample containing the virus composition and/or biological macromolecular components with the non-ionic organic polymer, applying the same to the hydrophilic membrane, allows a fast and reliable isolation of the virus composition and/or biological macromolecular components, e.g. whole virus particles and virus-like particles, from a material containing the same. That is, the method according to the present invention allows highly effective purification of whole virus particles, viral vectors and virus-like particles whereby the compounds required for conducting the method, namely, the non-ionic organic polymer and the hydrophilic membranes, are easily commercially available and, in addition are inexpensive; in particular, compared to the methods based on monolith chromatography columns.

Using the method according to the present invention achieved high product recoveries while reducing the level of contaminants. Further, the time for effecting the method is reduced compared to the time required for effecting the laborious method including SEC or other column based methods described in the art. Hence, the productivity can be significantly enhanced due to the easiness of the method according to the present invention.

As used herein, the term "virus" refers to whole virus also identified as virus particles and viral vector as well as virus like particles (VLPs) and viral proteins unless otherwise identified.

The term "biological macromolecular components" refers to biological macromolecules containing amino acid residues. The biological macromolecular components may be a single molecule, like a single protein, or may be macromolecular complexes, e.g. complexes of several subunits of a protein or different types of proteins. For example, the biological macromolecular components are molecules or complexes of at least 100 kDa size, like at least 150 kDa size. Examples of these components include immunoglobulins, enzymes, etc.

In comparison to SEC, no column packing needs to be performed and the productivity is much higher due to the higher flow rates achievable. The size of the required chromatography matrix is much smaller and the amount of sample that can be loaded onto the hydrophilic membrane is determined only by the capacity for the total virus compounds and/or biological macromolecular components.

In comparison to affinity matrices, either bead-based or membrane-based, the method according to the present invention is not limited by the availability of the affinity ligands. Affinity ligands can also be expensive to manufacture or to couple to an existing membrane. This is a reason for increasing production costs and hinders the possibility of using such a material for single use.

In ion exchange chromatography, product stability could be compromised by high concentrations of salt, and the virus product can be recovered in a particular buffer composition only.

Constrained co-hydration chromatography or steric exclusion chromatography based on monoliths work also with a size-based principle. However, although monoliths may have some advantages compared to bead based resins (e.g. high binding capacity, convective flow) they remain expensive, at least for vaccine manufacture. Hence, monoliths are preferentially not used for single use applications due to the high costs. Moreover, for multiple applications they require cleaning validation and the risk of process inconsistency due to impurity carryover.

A main advantage of the method according to the present invention are the low costs and high safety of the polymer and the membranes used, the high speed and productivity, and the potential of using it as a platform approach for any kind of virus compositions as well as biological macromolecular components, in particular, any kind of virus particles and VLPs since the separation is mainly based on size. Moreover, due to the easiness, and, in addition, due to the inexpensive material, the process can be adapted to be for single-use, thus, eliminating the need for cleaning validation, the risk of impurity carryover and product cross-contamination.

That is, the hydrophilic membranes are low cost hydrophilic membranes, e.g. the membranes are cellulose membranes, like regenerated cellulose membranes or reinforced membranes. In an embodiment, the hydrophilic membrane is a simple regenerated cellulose membrane being low in costs and easily available as well as easily processable.

In another embodiment, the virus composition is a whole virus particle or is a composition of virus like particles (VLPs) or is a mixture of two or more different whole viruses, or is a mixture of a whole virus particles and a virus like particle. The composition may be a composition of virus proteins and non-virus proteins. In an embodiment, the method for the purification is used for purifying virus particles or VLPs for later use in vaccines or other medical applications. The virus particles, VLPs, or macromolecules may be influenza virus, or yellow fever virus, or human papilloma virus, or vaccinia virus or adenovirus, or baculovirus, or hepatitis virus, or lentivirus, or poliovirus, or rabies virus, or rotavirus or rubella virus, particles or fragments thereof. In addition, the virus composition is a composition of viral vectors, e.g. useful in gene therapy or in vaccination.

The non-ionic organic polymer may be a non-ionic surfactant or non-ionic organic polymer having a hydrophobic part and a hydrophilic part.

In another embodiment, the non-ionic organic polymer is a polyol. That is, a polyol is a polyalcoholic compound containing hydroxylic groups, like an aliphatic polyether. Polyols may be linear or may be cyclic. In an embodiment, the polyols are oligo- or polymeric polyols based on short-chain alcohols. Examples of polyols are polyalkylene glycols or oligomeric glycerin. In an embodiment, the polyol is a polyethylene glycol or polypropylene glycol or mixtures thereof having an average molecular mass (also known as average molecular weight) of 100 to 100000 g/mol, for example, the polyethylene glycol or polypropylene glycol is a compound having an average molecular mass of 600 to 10000 g/mol.

In another embodiment, the non-ionic organic compound is a polyethylene glycol of PEG-600 to PEG-10000 including PEG 6000.

In another embodiment, the concentration of the non-ionic organic compound, for example in form of polyalkylene glycol including polyethylene glycol and polypropylene glycol in the sample contacted with the hydrophilic membrane is in the range of from 0.1 to 40 weight-%, e.g. 1 to 20 weight-%, like 2 to 10 weight-% based on the weight of the total sample.

That is, mixing the sample containing the virus composition and/or biological macromolecular components, in particular, containing the virus particles or VLPs, with the non-ionic organic polymer being selected from polyethylene glycol or polypropylene glycol or mixtures thereof, allows to efficiently purify the virus composition and/or biological macromolecular components with low costs and preferably without any further laborious cleaning steps. Since elution of the purified virus composition and/or biological macromolecular components is effective with a buffer containing a lower concentration or no non-ionic organic polymer, it may not be necessary to effect further process steps for eliminating the same from the sample.

Moreover, in an aspect of the present invention, the amount and size of the non-ionic organic polymer in form of the polyol, in particular, in form of the polyalkylene glycol, like polyethylene glycol or polypropylene glycol or mixtures thereof, depend primarily on the size and the shape of the virus particles or VLP to be purified. In this connection, it is preferred that in case of larger virus or larger VLP, smaller polyalkylene glycol (PAG), i.e. PAG, like PEG, with low average molecular mass, will be used, like PEG-600 to PEG-4000 while in case of smaller virus or VLPs, polyalkylene glycol with higher average molecular mass will be used.

Alternatively, the non-ionic organic polymer is a poloxamer.

Moreover, in case of larger virus or VLP, the concentration of the polyalkylene glycol, like the polyethylene glycol or polypropylene glycol or mixtures thereof is lower, e.g. in the range of from 1 to 10% (w/w) while with smaller virus or smaller VLP, the concentration of the polyalkylene glycol is higher.

Typically, 1 to 20 weight-%, like 2 to 12% (w/w) polyalkylene glycol, for example in form of polyethylene glycol, is added to the sample containing the virus composition. In case of biological macromolecular components, the amount of polymer added is in between 1 to 40 % (w/w) like 2 to 20 % (w/w).

Applying the method according to the present invention, it is possible to efficiently reduce the amount of contaminants in the sample, e.g. the amount of contaminating DNA. In an embodiment, the amount of DNA in the purified sample compared to the amount of DNA in the starting sample is reduced at least 50 %, e.g. at least 70%, like at least 80%, e.g. at least 90%, at least 95 %.

That is, typically, the contaminants including DNA and host cell proteins are present in the effluent and the washing buffer while the virus remains in or on the hydrophilic membrane. These viruses present in or on the hydrophilic membranes may be eluted with an elution buffer not containing any non-ionic organic polymer or containing a reduced amount of the non-ionic organic polymer compared to the sample or the washing buffer. In addition, the eluted virus compositions do not contain large amounts of non-ionic organic polymers, thus, reducing further steps accordingly.

Further, the present invention relates to the use of the method according to the present invention for isolating viruses or VLPs, in particular, vaccinia virus and influenza virus containing compositions for use in vaccines.

Other examples for suitable virus include: yellow fever, dengue virus, Chikungunya virus, polio virus, human papilloma virus, adenovirus, baculovirus, flavivirus, hepatitis virus, herpes simplex virus, japanese encephalitis virus, lentivirus, measles virus, mumps virus, poliovirus, rabies virus, rotavirus, rubella virus, Semliki forest virus, and where applicable VLPs of the corresponding viruses like human papilloma virus VLPs and influenza VLPs.

Finally, the present invention relates to the use of a kit for purification of viruses and/or VLPs. The kit contains non-ionic organic polymers as defined herein as well as a hydrophilic membrane. The kit is useful for single use only due to the low costs of the material. Thus, laborious cleaning of the equipment is not necessary.

The following examples illustrate the invention further without limiting the same to the specific embodiment described in the examples.

### Examples

### Example 1. Purification of adherent MDCK (MDCK_{adh}) cell culture-based influenza virus particles.

Clarified cell culture fluid (cCCF) containing influenza virus particles produced in adherent MDCK cells was used as starting material. Two different adsorption matrices were used: 1) a 0.34 mL monolith with hydroxyl (OH) ligands and 2) 75 cm² of regenerated cellulose (RC) membranes with a pore size of 1 µm and a diameter of 2.5 cm. The membranes were stacked inside a stainless steel filter holder. All experiments were performed on a Äkta liquid chromatography system (GE Healthcare Life Sciences, Uppsala, Sweden). Triplicate runs were performed for both adsorption matrices. The purification runs were conducted as follows: the matrix was equilibrated with binding buffer (phosphate buffer saline (PBS)) at a concentration of 8% PEG 6000. A volume of 8 mL of starting material was diluted 1:2 with a 16% (w/w) PEG 6000 stock solution to achieve a final PEG concentration of 8% (w/w) in the sample equal to that of the binding buffer; this mixture is defined as injected sample. After sample injection, the matrix was washed with binding buffer until stable UV absorbance was achieved. Desorption of influenza virus particles was carried out by flushing the matrix with PBS without PEG 6000. The hemagglutination activity (aHA) expressed as hemagglutination units (HAU) and DNA content were quantified in the injected sample, the flow through fraction and the collected product fraction. The results showed that at the same process conditions, about 5% of influenza virus is lost in the flow through when using the monolithic matrix. In the case of the RC membrane, HAU in the flow through was below the level of detection (LOD). The HAU recovery was about 71% and 87% for the monolith and RC membranes, respectively; this difference was found to be statistically significant with p=0.05.

### Example 2. Purification of suspension MDCK (MDCKₛᵤₛ) cell culture-based influenza virus particles.

The conditions of example 1 were repeated in RC membranes with cCCF containing influenza virus particles produced in suspension MDCK cells. HAU was below LOD in the flowthrough. The HAU recovery was 101 %.

The data obtained are shown in the table below and figure 1.

**Table 1. Relative recoveries and standard error of hemagglutination units (HAU) and DNA from purification of adherent (MDCK_{adh}) and suspension (MDCKₛᵤₛ) cell culture-based influenza virus particles.**

| Matrix/sample | HAU (%) FT | HAU (%) Elution | DNA (%) FT | DNA (%) Elution |
|---|---|---|---|---|
| Monolith/MDCK_{adh} | 5.39 ± 0.71 | 71.03 ± 3.61 | 90.77 ± 5.84 | 2.57 ± 0.16 |
| RC membrane/MDCK_{adh} | < LOD | 87.38 ± 1.83 | 78.89 ± 1.25 | 3.45 ± 0.08 |
| RC membrane/MDCKₛᵤₛ | < LOD | 100.56 ± 7.33 | 81.25 ± 0.68 | 0.63 ± 0.00 |

| | | | | |
|---|---|---|---|---|
| HAU = hemagglutination units, FT = flowthrough, RC = regenerated cellulose, LOD = limit of detection | | | | |

## Claims

1. A method for the purification of virus compositions and/or biological macromolecular components in a sample comprising the steps of:
- mixing the sample containing virus compositions and/or biological macromolecular components with a non-ionic organic polymer;
- contacting the mixture of the sample containing virus composition and/or biological macromolecular components and non-ionic organic polymer with a hydrophilic membrane;
- optionally washing the hydrophilic membrane with a washing solution containing a non-ionic organic polymer; and
- eluting the virus composition from the membrane with an elution solution containing no or a reduced amount of non-ionic polymer used for the binding for obtaining purified virus compositions and/or biological macromolecular components.

2. The method according to claim 1 wherein the virus compositions contains whole virus particles or virus like particles (VLPs) or viral gene vectors.

3. The method according to any one of the preceding claims wherein the hydrophilic membrane is a cellulose membrane, in particular, a regenerated and/or reinforced cellulose membrane.

4. The method according to any one of the preceding claims, wherein the non-ionic organic polymer is polyol or poloxamer.

5. The method according to any one of the preceding claims wherein the non-ionic organic polymer is a polyalkylene glycol, in particular, a polyethylene glycol or polypropylene glycol or mixtures thereof.

6. The method according to any one of the preceding claims, wherein the virus composition is virus particles or VLP and the non-ionic organic polymer is polyethylene glycol whereby the amount and size of the polyethylene glycol added depends mainly on the size of the virus particles or VLP to be purified.

7. The method according to any one of the preceding claims for the purification of different viruses or virus and particles from the same sample based on the amount and size of the non-ionic organic polymer, in particular, based on the amount and size of the polyalkylene glycol, like polyethylene glycol, added.

8. The method according to any one of the preceding claims wherein the non-ionic organic compound is a polyethylene glycol of PEG-600 to PEG-10000 and/or the PEG-concentration in the sample contacted with the hydrophilic membrane is in the range of from 0.1 to 40 % (w/w), preferably, 1 to 20 % (w/w) based on the sample.

9. The method according to any one of the preceding claims wherein the virus composition obtained is reduced in the amount of DNA compared to the starting sample of at least 50%.

10. Virus compositions and/or biological macromolecular components obtainable with the method according to any one of claims 1 to 9.

11. The virus composition according to claim 10 being a purified virus composition of either whole virus particles or VLPs.

12. The use of a method according to any one of claims 1 to 9 for isolating virus compositions including whole virus and VLP, in particular vaccinia virus and influenza virus containing compositions ready for use.

13. The use of a method according to any one of claims 1 to 9 for isolating biological macromolecular components, in particular, proteins like immunoglobulins.

14. The use of a kit for purification of virus compositions including virus and/or VLPs and/or biological macromolecular components, including non-ionic organic polymer as defined in any one of claims 1 to 8 and a hydrophilic membrane as defined in any one of claims 1 to 8.

15. The use of a kit according to claim 14 wherein the kit is single use.
